# NOUVEAU FASCICULE DE BREVET EUROPEEN

(11) **EP 2 198 849 B2**
(45) Date de publication et mention de la décision concernant l'opposition: **11.11.2020**
(45) Mention de la délivrance du brevet: 13.12.2017
(21) Numéro de dépôt: 09179895.9
(22) Date de dépôt: 18.12.2009
(51) Int. Cl.: A61K 8/37, A61K 8/41, A61K 8/42, A61Q 5/10

(54) **Composition de teinture d'oxydation des fibres kératiniques**
Oxidations-Färbezusammensetzung für Keratinfasern
Composition for oxidising dyeing of keratinous fibres

(30) Priorité: 19.12.2008 FR 0807288
(43) Date de publication de la demande: 23.06.2010
(73) Titulaire: L'Oréal, 75008 Paris (FR)
(72) Inventeur: Hercouet, Leïla, 93360, Neuilly Plaisance (FR); Simonet, Frédéric, 92110, Clichy (FR); Clement, Franck, 91700, Sainte Genevieve Des Bois (FR); Audousset, Marie-Pascale, 92600, Asnieres (FR); Schlosser, Isabelle, 75009, Paris (FR)
(74) Mandataire: L'Oreal

(56) Documents cités:
- EP-A- 0 166 100
- EP-A- 1 142 563
- EP-A- 1 762 222
- EP-A1- 0 474 118
- EP-A1- 0 593 038
- EP-A2- 1 726 289
- WO-A-97/12587
- WO-A-2005/055966
- WO-A1-2006/026851
- DE-A1-102006 020 050
- FR-A- 2 402 446
- FR-A- 2 870 724
- GB-A- 2 142 348
- GB-A- 2 188 948
- JP-A- 1 165 514
- JP-A- H09 249 537
- US-A- 3 100 739

## Description

La présente demande a pour objet une composition pour la teinture d'oxydation des fibres kératiniques.

Il est connu de teindre les fibres kératiniques et en particulier les cheveux humains avec des compositions tinctoriales contenant des colorants d'oxydation, en particulier des précurseurs de colorants d'oxydation et des modificateurs de coloration.

Les précurseurs de colorants d'oxydation, appelés généralement bases d'oxydation, sont initialement des composés incolores ou faiblement colorés qui, associés à des produits oxydants, peuvent donner naissance par un processus de condensation oxydative à des composés colorés et colorants. Ce sont en général des composés tels que les ortho- ou para-phénylènediamines, les ortho- ou para-aminophénols et les bases hétérocycliques.

On sait également que l'on peut faire varier les nuances obtenues avec ces bases d'oxydation en les associant à des coupleurs ou modificateurs de coloration, ces derniers étant généralement choisis parmi les méta-diaminobenzènes, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, appelée également coloration d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agressions extérieures telles que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui comporte en général des zones différemment sensibilisées (c'est-à-dire abîmées) de sa pointe à sa racine.

De nombreuses tentatives ont été faites dans le domaine de la coloration capillaire afin d'améliorer les propriétés tinctoriales à l'aide, par exemple, d'adjuvants. Cependant, le choix de ces adjuvants est délicat dans la mesure où ils doivent améliorer les propriétés tinctoriales des compositions tinctoriales sans nuire aux autres propriétés de ces compositions. En particulier, ces adjuvants ne doivent pas nuire aux propriétés d'éclaircissement des fibres kératiniques et les propriétés d'application de la coloration.

Le but de la présente invention est d'obtenir de nouvelles compositions pour la teinture d'oxydation des fibres kératiniques qui ne présentent pas les inconvénients de l'art antérieur. Plus particulièrement, le but de la présente invention est d'obtenir des compositions de coloration d'oxydation des fibres kératiniques, présentant des propriétés tinctoriales améliorées qui permettent d'atteindre l'éclaircissement souhaitée et qui soient faciles à mélanger et à appliquer, notamment qui ne coulent pas et restent bien localisées au point d'application. Par propriétés tinctoriales améliorées, on entend en particulier une amélioration au niveau de la puissance/intensité et/ou de l'homogénéité de la teinture.

Ainsi, ce but est atteint par la présente invention qui a pour objet une composition pour la teinture d'oxydation des fibres kératiniques, et en particulier des fibres kératiniques humaines telles que les cheveux, comprenant A) au moins 25 % en poids du poids total de la composition d'un ou plusieurs corps gras, au moins un des corps gras étant choisi parmi les amides gras et les esters d'acide gras solides à la température de 25°C et à pression atmosphérique (760 mm de Hg) et au moins un des corps gras est choisi parmi les corps gras liquides à la température de 25°C et à pression atmosphérique (760 mm de Hg), B) un ou plusieurs précurseurs de colorants, C) un ou plusieurs agents oxydants et D) un ou plusieurs agents alcalins, dans laquelle l'agent alcalin est une alcanolamine. Les documents GB 2188948 et FR 7727096 concernent des compositions pour teinture des cheveux. La composition conforme à la présente invention se distingue par ses propriétés tinctoriales améliorées. En particulier, la composition de l'invention conduit à des colorations qui présentent une bonne puissance et/ou intensité et/ou une bonne homogénéité de la couleur le long de la fibre entre la pointe et la racine des cheveux (appelé aussi la sélectivité de la coloration) et/ou une bonne chromaticité. La composition de l'invention s'applique sans difficulté sur les fibres kératiniques, sans couler. Cette composition permet aussi une plus faible dégradation des fibres kératiniques au cours du processus de coloration.

Enfin, les colorations obtenues à l'aide des compositions de l'invention sont tenaces, et résistent aux diverses agressions extérieures que peuvent subir les fibres kératiniques.

La présente invention a également pour objet un procédé de teinture des fibres kératiniques mettant en oeuvre la composition conforme à l'invention.

Ainsi que cela a été mentionné, la composition de l'invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0,1%). Ils présentent dans leur structure un enchaînement d'au moins deux groupements siloxane ou au moins une chaine hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène ou le décaméthylcyclopentasiloxane.

Selon un mode de réalisation particulier, la composition comprend au moins 25 % de corps gras différents des acides gras.

Parmi les corps gras présents, la composition de l'invention peut contenir un ou plusieurs amide gras d'une alcanolamine éventuellement subsituée et d'un acide gras en C9-C30. L'alcanolamine est par exemple une mono ou dialcanolamine en C2-C10, de préférence en C2-C4.

A titre d'exemple, l'amide gras utile dans la présente invention répond à la formule (I) suivante : 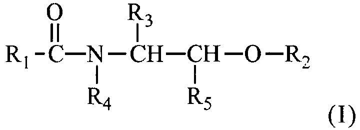 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, un radical hydroxyalkyle en C2-C6 ou un radical -CH₂-CHOH-CH₂-O-R₆ dans lequel R₆ désigne un radical hydrocarboné en C₁₀-C₂₆ ;
- R₅ désigne un atome d'hydrogène ou un radical hydrocarboné en C₁-C₄ mono ou polyhydroxylé.

Selon un mode de réalisation particulier, l'amide gras est un céramide, notamment un céramide de formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical α-hydroxyalkyle en C16

A titre d'exemple de céramide, on peut citer
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés, et mieux encore parmi la N-oléoyldihydrosphingosine, la N-2-hydroxypalmitoyl-dihydro-sphingosine et la N-stéaroylphytosphingosine.

Parmi les amides gras utiles dans la composition de l'invention, on peut citer en particulier les amides de mono- ou dialcanolamine et d'acide gras en C12-C30, de préférence en C₁₄-C₃₀, de préférence d'une alcanolamine en C₂-C₁₀, voire C₂-C₆ et d'un acide gras en C₁₄-C₂₂.

L'acide gras peut être saturé ou insaturé, linéaire ou ramifié.

A titre d'exemple d'amide d'une alcanolamine et d'un acide gras en C₁₂-C₃₀ (B), on peut citer :
- le diéthanolamide d'acide oléique, tel que l'amide commercialisé sous la dénomination commerciale MEXANYL® GT par la société CHIMEX,
- le monoéthanolamide d'acide myristique, tel que l'amide commercialisé sous la dénomination commerciale COMPERLAN® MM par la société COGNIS,
- le diéthanolamide d'acides gras de soja, tel que l'amide commercialisé sous la dénomination commerciale COMPERLAN® VOD par la société COGNIS,
- l'éthanolamide d'acide stéarique, tel que l'amide commercialisé sous la dénomination commerciale MONAMID® S par la société UNIQEMA,
- le monoisopropanolamide d'acide oléique, tel que l'amide commercialisé sous la dénomination commerciale WITCAMIDE® 61 par la société WITCO,
- le diéthanolamide d'acide linoléique, tel que l'amide commercialisé sous la dénomination commerciale PURTON® SFD par la société ZSCHIMMER SCHWARZ,
- le monoéthanolamide d'acide stéarique, tel que l'amide commercialisé sous la dénomination commerciale MONAMID® 972 par la société ICI/UNIQEMA,
- le monoéthanolamide d'acide béhénique, tel que l'amide commercialisée sous la dénomination commerciale INCROMIDE® BEM de CRODA,
- le monoisopropanolamide d'acide isostéarique, tel que l'amide commercialisé sous la dénomination commerciale WITCAMIDE® SPA par la société WITCO,
- le diéthanolamide d'acide érucique, tel que l'amide commercialisé sous la dénomination commerciale diéthanolamide d'acide érucique par la société STEARINERIES DUBOIS,
- le monoéthanolamide d'acide ricinoléique, tel que l'amide commercialisé sous la dénomination commerciale monoéthanolamide ricinoléique par la société STEARINERIES DUBOIS.
- le monoisopropanolamide d'acides de coprah, tel que l'amide commercialisé sous la dénomination commerciale EMPILAN® par la société HUNTSMAN
- le monoéthanolamide d'acides de coprah, tel que l'amide commercialisé sous la dénomination commerciale MONAMID C M A® par la société CRODA .

De préférence, l'amide gras est choisi parmi le monoéthanolamide d'acide stéarique, le monoisopropanolamide d'acides de coprah, le monoéthanolamide d'acides de coprah et la Noléoyldihydrosphingosine.

Selon une variante de l'invention, l'amide gras est un amide gras solide à température ambiante (25°C).

Selon un mode de réalisation particulier, la composition de l'invention comprend un ou plusieurs amides d'alcanolamine et d'acide gras en C14-C30 et un ou plusieurs céramides.

Parmi les corps gras présents, la composition peut contenir un ester d'acides gras en C8-C30.

Le ou les esters d'acide gras sont par exemple des mono- ou polyesters, de préférence choisis parmi les monoesters, diesters et triesters issus de la réaction de monoacides ou diacides, linéaires ou ramifiés, saturés ou insaturés, comportant de 8 à 30 atomes de carbone, éventuellement hydroxylés, avec des monoalcools ou des polyols, saturés ou insaturés, linéaires, ramifiés ou cycliques, comportant de 2 à 1000 atomes de carbone et de 1 à 30 groupement(s) hydroxyle.

Les acides gras considérés sont par exemple l'acide stéarique, l'acide palmitique, l'acide laurique, l'acide oléique, l'acide myristique.

Les monoalcools ou polyols considérés sont par exemple l'éthanol, l'isopropanol, l'isooctanol, le dodécanol, l'alcool stéarylique, l'éthylène glycol, le propylène glycol, le glycérol, les polyéthylène glycols, les polypropylène glycols, le glucose, le méthyl glucose, le sorbitol, l'anhydride de sorbitol, le pentaérythrytol.

Les monoalcools et polyols, qui ne sont pas des polyéthylèneglycols et/ou des polypropylèneglycols peuvent être éventuellement polyoxyalkylénés, et plus particulièrement polyoxyéthylénés et/ou polyoxypropylénés, le nombre de moles d'oxyde d'éthylène et/ou d'oxyde de propylène par mole d'ester étant alors de préférence compris entre 2 et 400, mieux entre 2 et 200.

De préférence, les monoalcools ou polyols, s'ils sont différents des polyéthylèneglycols et/ou des polypropylèneglycols, ne sont pas polyoxyalkylénés.

Le ou les ester(s) d'acide gras utilisable(s) selon l'invention sont généralement non ioniques, c'est-à-dire qu'ils ne comportent pas de charges ioniques.

A titre d'exemples d'esters pouvant être mis en oeuvre selon l'invention, on peut citer le myristate d'isopropyle, les stéarate, myristate ou palmitate de stéaryle, les mono- ou distéarate d'éthylène glycol, les mono- ou distéarate de polyéthylène glycols tels que le PEG-40 stéarate, le monopalmitate de sorbitane, l'isostéarate de glycéryle, le dipélargonate de propylèneglycol, le palmitate de 2-éthylhexyle, le tristéarate de sorbitane, le sébacate de di(2-éthylhexyle), le trihydroxystéarate de glycéryle, les stéarate, palmitate ou myristate de cétyle, les stéarate, palmitate ou myristate de myristyle, l'isononanoate d'isononyle.

Selon un mode de réalisation particulier, l'ester est un ester d'acide gras et d'alcool gras, l'acide et l'alcool comprenant de 6 à 30 atomes de carbone.
- A titre d'ester d'acide gras, on peut citer les esters et diesters de sucres d'acides gras. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61% de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

De préférence, le ou les esters d'acides gras sont choisis parmi, le distéarate d'éthylène glycol, les stéarate, palmitate ou myristate de stéaryle, les stéarate, palmitate ou myristate de cétyle, les stéarate, palmitate ou myristate de myristyle.

Selon une variante, l'ester d'acide gras est un ester gras solide à température ambiante.

La concentration en amide gras et/ou en ester(s) d'acide gras est en général comprise entre 0,01 et 50% en poids, en particulier entre 0,1 et 30 % en poids, et de manière plus préférée entre 0,2 et 10 % en poids, par rapport au poids total de la composition.

La composition de l'invention peut contenir d'autres corps gras pour parvenir à la quantité requise d'au moins 25 %. La composition selon l'invention présente plus particulièrement une teneur totale en corps gras allant de 25 à 85% en poids, encore plus préférentiellement de 25 à 65%, mieux de 30 à 55% en poids par rapport au poids total de la composition.

Les corps gras autres que les amides gras et les esters d'acides gras précédemment définis sont notamment choisis parmi les alcanes inférieurs, les alcools gras, les esters d'alcool gras, les huiles non siliconées en particulier les huiles minérales, végétales, animales ou synthétiques, les cires non siliconées et les silicones.

Il est rappelé qu'au sens de l'invention, les corps gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs, ces derniers comprennent de 6 à 30 atomes de carbone et sont linéaires ou ramifiés, éventuellement cycliques. A titre d'exemple, les alcanes peuvent être choisis parmi l'hexane et le dodécane, les isoparaffines comme l'isohexadécane et l'isodécane.

Comme huiles non siliconées utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, la vaseline, l'huile de vaseline, les polydécènes, le polyisobutène hydrogéné tel que Parléam®.
- les huiles fluorées; on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables dans la composition de l'invention sont non oxyalkylénés. Il sont saturés ou insaturés, linéaires ou ramifiés, et comportent 6 à 30 atomes de carbone et plus particulièrement de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires non siliconées susceptibles d'être utilisées dans la composition de l'invention sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général

Les esters autres que ceux définis précédemment sont les esters de mono ou polyacides aliphatiques saturés ou insaturés, linéaires ou ramifiés en C₁-C₇ et de mono ou polyalcools aliphatiques saturés ou insaturés, linéaires ou ramifiés en C₁-C₂₆, le nombre total de carbone des esters étant plus particulièrement supérieur ou égal à 10.

Parmi les monoesters, on peut citer le; le lactate de cétyle ; le lactate d'alkyle en C₁₂-C₁₅ ; le lactate d'isostéaryle ; le lactate de lauryle ; le lactate de linoléyle ; le lactate d'oléyle ;

Toujours dans le cadre de cette variante, on peut également utiliser les esters d'acides di ou tricarboxyliques en C₄-C₇ et d'alcools en C₁-C₂₂ et les esters d'acides mono di ou tricarboxyliques en C₄-C₇ et d'alcools di, tri, tétra ou pentahydroxy en C₂-C₂₆.

On peut notamment citer ÷ l'adipate de diisopropyle ; l'adipate de di n-propyle ; l'adipate de dioctyle ; l'adipate de diisostéaryle ; le maléate de dioctyle; le citrate de triisopropyle ; le citrate de triisotéaryle ; trilactate de glycéryle ; ; le citrate de trioctyldodécyle ; le citrate de trioléyle,

Parmi les esters cités ci-dessus, on préfère utiliser le malate de dioctyle.

Les corps gras peuvent comprendre des silicones. Les silicones utilisables dans la composition de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 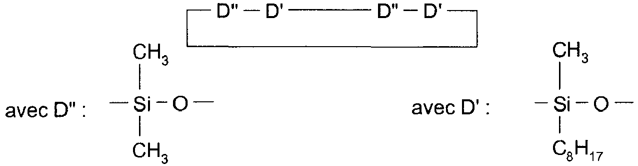 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy)bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

Plus particulièrement, les corps gras autres que les amides gras et les esters d'acide gras précédemment définis sont choisis parmi corps gras liquides à la température de 25°C et à la pression atmosphérique.

Selon cette variante, le ou les corps gras liquides additionnels sont choisis parmi l'huile de vaseline, les polydécènes, les esters d'acides gras ou d'alcools gras, liquides ou leurs mélanges, en particulier, le ou les corps gras de la composition selon l'invention sont non siliconés.

La composition selon l'invention comprend un ou plusieurs précurseurs de colorants.

Ce ou ces précurseurs de colorants sont choisis parmi les bases d'oxydation et les coupleurs.

La ou les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques ainsi que les sels d'addition de ces composés avec un acide.

Ces bases d'oxydation peuvent être en particulier cationiques.

Les para-phénylènediamines utilisables dans le cadre de l'invention peuvent notamment être choisies parmi les composés de formule (II) suivante et leurs sels d'addition avec un acide : 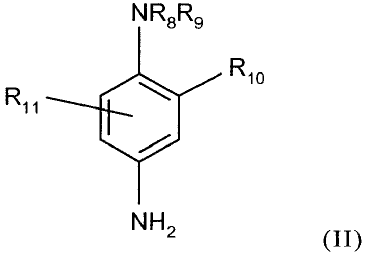 dans laquelle :
▪ R₈ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
▪ R₉ représente un atome d'hydrogène, un radical alkyle en C₁-C₄, monohydroxyalkyle en C1-C4, polyhydroxyalkyle en C₂-C₄, alcoxy(C₁-C₄)alkyle(C₁-C₄) ou alkyle en C₁-C₄ substitué par un groupement azoté ;
▪ R₈ et R₉ peuvent également former avec l'atome d'azote qui les porte un hétérocycle azoté à 5 ou 6 chaînons éventuellement substitué par un ou plusieurs groupements alkyle, hydroxy ou uréido ;
▪ R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
▪ R₁₁ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les para-phénylènediamines de formule (II) ci-dessus, on peut plus particulièrement citer la para-phénylènediamine, la para-toluylènediamine, la 2-chloro-para-phénylènediamine, la 2,3-diméthyl-para-phénylènediamine, la 2,6-diméthyl-para-phénylènediamine, la 2,6-diéthyl-para-phénylènediamine, la 2,5-diméthyl-para-phénylènediamine, la N,N-diméthyl-para-phénylènediamine, la N,N-diéthyl-para-phénylènediamine, la N,N-dipropyl-para-phénylènediamine, la 4-amino-N,N-diéthyl-3-méthyl-aniline, la 4-N,N-bis-(β-hydroxyéthyl)amino-2-méthyl-aniline, la N,N bis β-hydroxyéthyl paraphénylènediamine, la 4-N,N-bis-(β-hydroxyéthyl)-amino-2-chloro-aniline, la 2-β-hydroxyéthyl-para-phénylènediamine, la 2-fluoro-para-phénylènediamine, la 2-isopropyl-para-phénylènediamine, la N-(β-hydroxypropyl)-para-phénylènediamine, la 2-hydroxyméthyl-para-phénylènediamine, la N,N-diméthyl-3-méthyl-para-phénylènediamine, la N,N-(éthyl,β-hydroxyéthyl)-para-phénylènediamine, la N-(β,γ-dihydroxypropyl)-para-phénylènediamine, la N-(4'-aminophényl)-para-phénylènediamine, la N-phényl-para-phénylènediamine, la 2-β-hydroxyéthyloxy-para-phénylènediamine, la 2-β-acétylaminoéthyloxy-para-phénylènediamine, la N-(β-méthoxyéthyl)-para-phénylènediamine, la 2-méthyl-1-N-β-hydroxyéthyl-para-phénylènediamine, et leurs sels d'addition avec un acide.

Parmi les para-phénylènediamines de formule (II) ci-dessus, on préfère tout particulièrement la para-phénylènediamine, la para-toluylènediamine, la 2-isopropyl-para-phénylènediamine, la 2-β-hydroxyéthyl-para-phénylènediamine, la 2,6-diméthyl-para-phénylènediamine, la 2,6-diéthyl-para-phénylènediamine, la 2,3-diméthyl-para-phénylènediamine, la 2-chloro-para-phénylènediamine, la N,N bis β-hydroxyéthyl paraphénylènediamine et leurs sels d'addition avec un acide.

On utilisera tout particulièrement la para-phénylènediamine et la para-toluylènediamine, la N,N bis β-hydroxyéthyl paraphénylènediamine et leurs sels d'addition avec un acide.

Selon l'invention, on entend par bases doubles les composés comportant au moins deux noyaux aromatiques sur lesquels sont portés des groupements amino et/ou hydroxyle.

Parmi les bases doubles utilisables à titre de bases d'oxydation dans la composition conforme à l'invention, on peut notamment citer les composés répondant à la formule (III) suivante et leurs sels d'addition avec un acide : 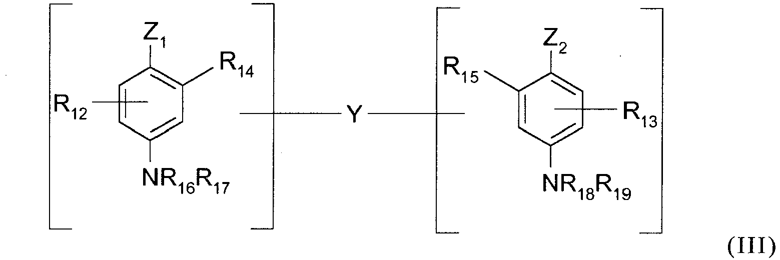 dans laquelle :
▪ Z₁ et Z₂, identiques ou différents, représentent un radical hydroxyle ou -NH₂ pouvant être substitué par un radical alkyle en C₁-C₄ ou par un bras de liaison Y ;
▪ le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
▪R₁₂ et R₁₃ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
▪ R₁₄, R₁₅, R₁₆, R₁₇, R₁₈ et R₁₉, identiques ou différents, représentent un atome d'hydrogène, un bras de liaison Y ou un radical alkyle en C₁-C₄ ;
▪ étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

Les para-aminophénols utilisables dans le cadre de l'invention peuvent notamment être choisis parmi les composés répondant à la formule (IV) suivante et leurs sels d'addition avec un acide : 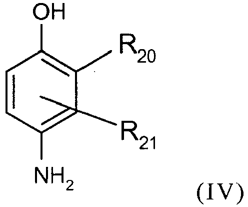 dans laquelle :
▪ R₂₀ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄ ;
▪ R₂₁ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

Le para-aminophénol et le 4-amino-3-méthyl-phénol sont encore plus préférés.

Les ortho-aminophénols utilisables à titre de bases d'oxydation dans le cadre de la présente l'invention sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

Parmi les bases hétérocycliques utilisables à titre de bases d'oxydation dans la composition conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol ; le 2-[(3-amino-pyrazolo[1,5-a]pyrimidin-7-yl)-(2-hydroxy-éthyl)-amino]-éthanol ; le 2-[(7-amino-pyrazolo[1,5-a]pyrimidin-3-yl)-(2-hydroxy-éthyl)-amino]-éthanol ; la 5,6-diméthyl-pyrazolo-[1,5-a]-pyrimidine-3,7-diamine ; la 2,6-diméthyl-pyrazolo-[1,5-a]-pyrimidine-3,7-diamine ; la 2,5,N7,N7-tétraméthyl-pyrazolo-[1,5-a]-pyrimidine-3,7-diamine ; la 3-amino-5-méthyl-7-imidazolylpropylamino-pyrazolo-[1,5-a]-pyrimidine ; leurs sels d'addition et leurs formes tautomères, lorsqu'il existe un équilibre tautomérique.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme les 4,5-diaminopyrazoles tels que par exemple le 4,5-diamino-1-méthyl-pyrazole, le 4,5-diamino-1-(4'-chlorobenzyl)-pyrazole, le 4,5-diamino-1,3-diméthyl-pyrazole, le 4,5-diamino-3-méthyl-1-phényl-pyrazole, le 4,5-diamino-1-méthyl-3-phényl-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl-pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole et le 4-amino-5-(2'-aminoéthyl)amino-1,3-diméthyl-pyrazole ; le 3,4-diamino-pyrazole ; le 4-amino-1,3-diméthyl-5-hydrazino-pyrazole ; les 3,4,5-triaminopyrazoles tels que par exemple le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole ; et leurs sels d'addition avec un acide.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels d'addition.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels d'addition.

A titre de bases d'oxydation cationiques utilisables dans les compositions selon l'invention, on peut citer par exemple les composés suivants : les para-phénylènediamines telles que notamment décrites dans les demandes de brevets FR-A-2 766 177 et FR-A-2 766 178, les para-aminophénols tels que décrits par exemple dans les demandes de brevet FR-A-2 766 177 et FR-A-2 766 178, les ortho-phénylènediamines telles que décrites par exemple dans les demandes de brevet FR-A-2 782 718, FR-A-2 782 716 et FR-A-2 782 719, des ortho-aminophénols ou des bases doubles cationiques telles que des dérivés de type bis(aminophényl)alkylènediamine décrites dans les demandes de brevet FR-A-2 766 179, ainsi que les bases hétérocycliques cationiques, ces composés portant au moins un atome d'azote quaternaire.

De préférence, les bases d'oxydation cationiques utilisables dans les compositions selon l'invention sont des para-phénylènediamines cationiques.

De manière avantageuse, une variante consiste à mettre en oeuvre des bases d'oxydation cationiques de structure para-phénylènediamine, dont au moins une des fonctions amine est une amine tertiaire porteuse d'un noyau pyrrolidinique, la molécule possédant au moins un atome d'azote quaternisé. De telles bases sont, par exemple, décrites dans le document EP-A-1 348 695.

La composition selon l'invention comprend de préférence une quantité totale de bases d'oxydation allant de 0,0005 à 12 % en poids par rapport au poids total de la composition. De préférence, elle comprend une quantité totale de bases d'oxydation allant de 0,005 à 8 % en poids, et mieux encore de 0,05 à 5 % en poids, par rapport au poids total de ladite composition.

Le ou les coupleurs utilisables dans la composition selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que, par exemple, les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La composition selon l'invention comprend généralement une quantité totale de coupleurs allant de 0,0001 à 15 % en poids par rapport au poids total de la composition. De préférence, elle comprend une quantité totale de coupleurs allant de 0,001 à 10 % en poids, et mieux encore de 0,01 à 8 % en poids, par rapport au poids total de la composition.

Les bases d'oxydation et coupleurs peuvent être présents dans les compositions de l'invention, sous forme de sels d'addition, et en particulier sous forme de sels d'addition avec un acide.

Les sels d'addition avec un acide utilisables dans le cadre de l'invention sont, notamment, choisis parmi les chlorhydrates, les bromhydrates, les sulfates, les citrates, les succinates, les tartrates, les lactates, les acétates, les alkylsulfates et les alkylsulfonates.

Lorsque les bases d'oxydation ou les coupleurs contiennent une ou plusieurs fonctions acide carboxylique ou sulfonique, des sels d'addition avec une base sont envisageables. Les sels d'addition avec une base utilisables dans le cadre des compositions tinctoriales de l'invention sont alors notamment ceux obtenus avec de la soude, de la potasse, de l'ammoniaque ou des aminés.

Selon un mode de réalisation particulier de l'invention, la composition comprend une ou plusieurs bases d'oxydation et un ou plusieurs coupleurs.

Selon une variante, la base d'oxydation additionnelle est choisie parmi les para-aminophénols, les bases hétérocycliques ainsi que les sels d'addition avec un acide correspondant.

La composition conforme à la présente invention comprend un ou plusieurs agents oxydants.

Un tel agent oxydant est choisi par exemple parmi les peroxydes tels que le peroxyde d'hydrogène, le peroxyde d'urée, les bromates ou ferricyanures de métaux alcalins, les persels tels que les perborates, percarbonates et les persulfates. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40 volumes.

La concentration en agent(s) oxydant(s) de la composition de l'invention va de préférence de 0.1 à 20% mieux de 0.5 à 10% du poids total de la composition.

La composition de l'invention comprend un ou plusieurs agents alcalins qui est une alcanolamine. Ce ou ces alcanolamines sont par exemple telles que les mono-, di- et triéthanolamines ainsi que leurs dérivés.

Lorsque la composition contient plusieurs agents alcalins dont une alkanolamine et de l'ammoniaque ou un de ses sels, la ou les amines organiques sont de préférence majoritaire en poids par rapport à la quantité ammoniac.

Selon un mode de réalisation particulier, la composition contient une faible quantité d'ammoniaque, voire pas d'ammoniaque. Selon ce mode de réalisation, la composition contient de préférence la monoéthanolamine.

La concentration en agent(s) alcalin(s) de la composition de l'invention va de préférence de 0.01 à 30%, et encore plus préférentiellement de 0.1 à 20% du poids total de la composition.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques, et leurs sels d'addition. Ces colorants directs peuvent être de nature non- ionique, anionique ou cationique.

La composition peut aussi contenir d'autres composés constituant le milieu de coloration. Ce milieu de coloration comprend généralement de l'eau ou un mélange d'eau et d'un ou plusieurs solvant(s) organique(s) acceptable(s), de préférence hydrosolubles sur le plan cosmétique.

A titre d'exemples de solvants organiques, on peut notamment citer les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, l'hexylène glycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,01 à 35 % en poids et, de préférence, entre environ 0,1 et 25 % en poids par rapport au poids total de la composition.

De préférence la composition de l'invention contient de l'eau. Encore plus préférentiellement la concentration en eau peut aller de 10 à 70%, mieux de 20 à 55% du poids total de la composition.

La composition conforme à l'invention peut contenir en outre un ou plusieurs adjuvant(s) utilisé(s) classiquement dans les compositions pour la teinture des cheveux.

Par "adjuvant", on entend un additif, différent des composés précités.

A titre d'exemples d'adjuvants utilisables, on peut citer les agents tensio-actifs anioniques, cationiques, non-ioniques, amphotères, zwittérioniques ou leurs mélanges ; les polymères anioniques, cationiques, non-ioniques, amphotères, zwittérioniques, les agents épaississants minéraux ou organiques, et en particulier les épaississants associatifs polymériques anioniques, cationiques, non-ioniques et amphotères, autres que les celluloses associatives selon l'invention ; les agents antioxydants ou réducteurs ; les agents de pénétration ; les agents séquestrants ; les parfums ; les tampons ; les agents dispersants ; les agents conditionneurs tels que par exemple les silicones volatiles ou non volatiles, modifiées ou non modifiées ; les agents filmogènes ; les céramides ; les agents conservateurs ; les agents opacifiants ; et les agents anti-statique.

Les adjuvants ci-dessus sont en général présents en quantité comprise pour chacun d'eux entre 0,01 et 20 % en poids par rapport au poids de la composition tinctoriale.

De préférence la composition de l'invention contient un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentent un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, en particulier C₁₈-C₃₀, oxyéthylénés.

A titre d'exemple de tensioactifs non ioniques mono- ou poly-glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition de l'invention est un tensioactif non ionique.

La teneur en tensioactifs dans la composition de l'invention représente plus particulièrement de 0,1 à 50 % en poids, de préférence de 0,5 à 30 % en poids par rapport au poids de la composition.

Bien entendu, l'homme de l'art veillera à choisir le(s) éventuel(s) adjuvant(s) mentionné(s) ci-avant(s), de manière telle que les propriétés avantageuses attachées intrinsèquement aux compositions selon l'invention ne soient pas, ou substantiellement pas, altérées par l'adjonction (les adjonctions) envisagée(s).

Le pH de la composition conforme à l'invention est généralement compris entre 3 et 12 environ, de préférence entre 5 et 11 environ, préférentiellement 7 à 11. Il peut être ajusté à la valeur désirée au moyen d'agents acidifiants ou alcalinisants habituellement utilisés en teinture des fibres kératiniques ou bien encore à l'aide de systèmes tampons classiques.

Les agents alcalins sont par exemple ceux décrits précédemment.

Parmi les agents acidifiants, on peut citer, à titre d'exemple, des acides minéraux ou organiques comme l'acide chlorhydrique, l'acide ortho-phosphorique, des acides carboxyliques comme l'acide tartrique, l'acide citrique, l'acide lactique, ou des acides sulfoniques.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment. La couleur peut être révélée à pH acide, neutre ou alcalin et l'agent oxydant peut être ajouté juste au moment de l'emploi ou il peut être mis en oeuvre simultanément ou séquentiellement aux autres composés de la composition de l'invention.

Après un temps de pose variant généralement de 1 à 60 minutes environ, de préférence 5 à 45 minutes environ, les fibres kératiniques sont rincées, éventuellement lavées au shampooing et rincées à nouveau, puis séchées.

La composition selon l'invention peut résulter du mélange d'au moins deux compositions et de préférence de 2 ou 3 compositions dont préférentiellement une composition oxydante comprenant au moins un agent oxydant tel que défini précédemment. Une des compositions peut être anhydre.

L'invention peut être mise en œuvre avec un dispositif à plusieurs compartiments ou "kit" de teinture dans lequel un premier compartiment contient une composition comprenant le ou les esters ou et/ou amides gras de l'invention associés éventuellement à d'autres corps gras additionnels, un second compartiment comprenant le ou les précurseurs de colorants et le ou les éventuels agents alcalins et un troisième compartiment comprenant le ou les agents oxydants, ce troisième compartiment pouvant contenir une partie des corps gras. Dans ce mode de réalisation, la composition comprenant le ou les esters ou et/ou amides gras de l'invention associés éventuellement à d'autres corps gras additionnels peut être anhydre. On entend, par composition anhydre, au sens de l'invention, une composition cosmétique présentant une teneur en eau inférieure à 5 % en poids, de préférence inférieure à 2% en poids et de manière encore plus préférée inférieure à 1% en poids par rapport au poids de ladite composition. Il est à noter qu'il s'agit plus particulièrement d'eau liée, comme l'eau de cristallisation des sels ou des traces d'eau absorbée par les matières premières utilisées dans la réalisation des compositions selon l'invention.

Selon un second mode de réalisation, le dispositif comprend un premier compartiment contenant une composition comprenant le ou les esters ou et/ou amides gras de l'invention associés éventuellement à d'autres corps gras additionnels et un ou plusieurs agents oxydants et un second compartiment contenant une composition comprenant le ou les précurseurs de colorants et éventuellement un ou plusieurs agents alcalins. Ce dispositif peut être équipé d'un moyen permettant de délivrer sur les cheveux le mélange souhaité, tel que les dispositifs décrits dans le brevet FR-A-2 586 913 au nom de la Demanderesse.

Selon un troisième mode de réalisation, le dispositif comprend un premier compartiment contenant une composition comprenant le ou les esters ou et/ou amides gras de l'invention aasociés éventuellement à d'autres corps gras additionnels, le ou les précurseurs de colorants, et éventuellement le ou les agents alcalins et un second compartiment contenant un ou plusieurs agents oxydants.

Les exemples qui suivent sont destinés à illustrer l'invention sans pour autant en limiter la portée.

### EXEMPLES

Les compositions suivantes ont été préparées :

### Exemple 1

| **Composition 1** | **Concentration (g%)** |
|---|---|
| DISTEARDIMONIUM HECTORITE | 3 |
| OCTYLDODECANOL | 11,5 |
| GLYCOL DISTEARATE | 8 |
| HUILE DE VASELINE | 64,488 |
| PROPYLENE CARBONATE | 1 |
| LAURETH-2 | 1 |
| POLYSORBATE 21 | 11 |
| N-OLEYL DI-HYDROSPHINGOSINE | 0,012 |

| **Composition 2** | **concentration (g%)** |
|---|---|
| ACIDE DIETHYLENE TRIAMINE PENTACETIQUE, SEL PENTASODIQUE EN SOLUTION AQUEUSE A 40 % | 1 |
| METABISULFITE DE SODIUM | 0,7 |
| MONOETHANOLAMINE | 14,5 |
| 1-METHYL-2,5-DIAMINOBENZENE | 2,25 |
| 2,4-DIAMINOPHENOXYETHANOL HCl | 0,05 |
| 1,3-DIHYDROXYBENZENE (RESORCINOL) | 2 |

| (suite) | |
|---|---|
| **Composition 2** | **concentration (g%)** |
| m-AMINOPHENOL | 0,36 |
| NATROSOL 250 HHR (hydroxyethylcellulose) | 1,5 |
| HEXYLENE GLYCOL | 3 |
| DIPROPYLENE GLYCOL | 3 |
| ALCOOL ETHYLIQUE | 8,25 |
| PROPYLENEGLYCOL | 6,2 |
| ACIDE ASCORBIQUE | 0,25 |
| EAU | Qs 100 g |

| **Composition 3** | **Concentration (g%)** |
|---|---|
| ACIDE DIETHYLENE TRIAMINE PENTACETIQUE, SEL PENTASODIQUE EN SOLUTION AQUEUSE A 40 % | 0,15 |
| PEROXYDE D'HYDROGENE EN SOLUTION A 50 % (EAU OXYGENEE 200 VOL.) | 12 |
| STANNATE DE SODIUM | 0,04 |
| PYROPHOSPHATE DE SODIUM | 0,03 |
| HUILE DE VASELINE | 20 |
| HEXADIMETHRINE CHLORIDE (MA à 60% ans l'eau) | 0,25 |
| POLYQUATERNIUM-6 (MA à 40% dans l'eau) | 0,5 |
| GLYCERINE | 0,5 |
| ALCOOL CETYLSTEARYLIQUE (C16/C18 30/70) | 8 |
| ALCOOL CETYLSTEARYLIQUE OXYETHYLENE (33 OE) | 3 |
| AMIDE D'ACIDES DE COLZA OXYETHYLENE (4 OE) PROTEGE à 92.3 % dans l'eau | 1,3 |
| VITAMINE E | 0,1 |
| ACIDE PHOSPHORIQUE | Qs pH 2.2 |
| EAU | QS 100 g |

### Exemple 2

| **Composition 1** | **Concentration (g%)** |
|---|---|
| DISTEARDIMONIUM HECTORITE | 3 |
| OCTYLDODECANOL | 11,5 |
| GLYCOL DISTEARATE | 4 |
| HUILE DE VASELINE | 64,488 |
| PROPYLENE CARBONATE | 1 |
| LAURETH-2 | 1 |
| POLYSORBATE 21 | 11 |
| STEARAMIDE MEA(AND) MONOETHANOLAMINE (AND) STEARIC ACID (96/2/2) | 4 |
| N-OLEYL DI-HYDROSPHINGOSINE | 0,012 |

| **Composition 2** | **concentration (g%)** |
|---|---|
| ACIDE DIETHYLENE TRIAMINE PENTACETIQUE, SEL PENTASODIQUE EN SOLUTION AQUEUSE A 40 % | 1 |
| METABISULFITE DE SODIUM | 0,7 |
| MONOETHANOLAMINE | 14,5 |
| 1-METHYL-2,5-DIAMINOBENZENE | 1,69 |
| 2-METHYL-5-HYDROXYETHYLAMINOPHENOL | 4,176 |
| 1-METHYL-2-HYDROXY-4-AMINO-BENZENE | 1,392 |
| 1,3-DIHYDROXYBENZENE (RESORCINOL) | 0,884 |
| p-AMINOPHENOL | 2,436 |
| NATROSOL 250 HHR (hydroxyethylcellulose) | 1,5 |
| DIPROPYLENE GLYCOL | 10 |
| ALCOOL ETHYLIQUE | 15 |
| PROPYLENEGLYCOL | 5 |
| ACIDE ASCORBIQUE | 0,25 |
| EAU | Qs 100 g |

La composition 3 mise en oeuvre dans cet exemple est identique à celle décrite à l'exemple 1.

Les compositions 1,2 et 3 de chacun des exemples sont mélangées au moment de l'emploi dans les proportions suivantes : 10 g de la composition 1 avec 4 g de la composition 2 et 16 g de la composition 3. Le mélange est appliqué sur des mèches de cheveux gris naturels à 90 % de cheveux blancs à raison de 10 g de mélange pour 1 g de cheveux. Après 30 min de pause, les cheveux sont rincés, lavés avec un shampooing standard et séchés.

La coloration capillaire est évaluée de manière visuelle.

| | |
|---|---|
| **Exemple 1** | Châtain clair naturel |
| **Exemple 2** | Châtain foncé à reflet rouge acajou |

## Revendications

1. Composition pour la teinture d'oxydation des fibres kératiniques, comprenant A) au moins 25 % en poids du poids total de la composition d'un ou plusieurs corps gras, au moins un des corps gras étant choisi parmi les amides gras et les esters d'acide gras solides à la température de 25°C et à pression atmosphérique (760 mm de Hg) et au moins un des corps gras est choisi parmi les corps gras liquides à la température de 25°C et à pression atmosphérique (760 mm de Hg), B) un ou plusieurs précurseurs de colorants, C) un ou plusieurs agents oxydants et D) un ou plusieurs agents alcalins dans laquelle l'agent alcalin est une alcanolamine.

2. Composition selon la revendication 1 **caractérisée en ce que** les amides gras sont des amides d'une mono ou dialcanolamine en C2-C10 éventuellement sustituée, et d'un acide gras en C9-C30.

3. Composition selon l'une quelconque des revendications précédentes dans laquelle l'amide gras correspond à la formule (I) suivante : 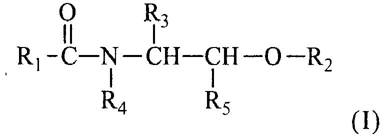 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, un radical hydroxyalkyle en C2-C6 ou un. radical -CH₂-CHOH-CH₂-O-R₆ dans lequel R₆ désigne un radical hydrocarboné en C₁₀-C₂₆ ;
- R₅ désigne un atome d'hydrogène ou un radical hydrocarboné en C₁-C₄ mono ou polyhydroxylé.

4. Composition selon la revendication 3 dans laquelle l'amide gras est un céramide de formule(I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical α-hydroxyalkyle en C16.

5. Composition selon l'une quelconque des revendications précédentes, **caractérisée en ce que** l'amide gras est choisi parmi le monoéthanolamide d'acide stéarique, le monoisopropanolamide d'acides de coprah, le monoéthanolamide d'acides de coprah et la Noléoyldihydrosphingosine.

6. Composition selon l'une quelconque des revendications précédentes dans laquelle l'ester d'acide gras est obtenu à partir d'acides gras en C8-C30.

7. Composition selon la revendication 6 dans laquelle l'acide gras est choisi parmi l'acide stéarique, l'acide palmitique, l'acide laurique, l'acide oléique, l'acide myristique.

8. Composition selon l'une quelconque des revendications précédentes dans laquelle l'ester est choisi parmi le distéarate d'éthylène glycol, les stéarate, palmitate ou myristate de stéaryle, les stéarate, palmitate ou myristate de cétyle, les stéarate, palmitate ou myristate de myristyle.

9. Composition selon l'une quelconque des revendications précédentes, **caractérisée en ce que** a concentration en amide gras et/ou en ester(s) d'acide gras est en général comprise entre 0,01 et 50% en poids, en particulier entre 0,1 et 30 % en poids, et de manière plus préférée entre 0,2 et 10 % en poids, par rapport au poids total de la composition.

10. Composition selon l'une quelconque des revendications précédentes dans laquelle le ou les corps gras liquides sont choisis parmi l'huile de vaseline, les polydécènes, les esters d'acide gras et d'alcool gras ou leur mélange.

11. Composition selon l'une quelconque des revendications précédentes, **caractérisée en ce que** le précurseur de colorant est choisi parmi les bases d'oxydation et les coupleurs.

12. Composition selon la revendication précédente, **caractérisée en ce que** le précurseur de colorant est choisi parmi les bases d'oxydation ortho- et para-phénylènediamines, les bases doubles, les ortho- et para-aminophénols, les bases hétérocycliques, ainsi que les sels d'addition de ces composés avec un acide et les coupleurs méta-aminophénols, les méta-phénylènediamines, les métadiphénols, les naphtols, les coupleurs hétérocycliques et les sels d'addition de ces composés avec un acide.

13. Composition selon l'une quelconque des revendications précédentes, **caractérisée en ce que** l'agent oxydant est un peroxyde, de préférence le peroxyde d'hydrogène.

14. Composition selon l'une quelconque des revendications précédentes dans laquelle l'agent alcalin est la monoéthanolamine.

15. Procédé de teinture des fibres kératiniques, **caractérisé en ce qu'**une composition telle que définie à l'une quelconque des revendications 1 à 14 est appliquée sur les fibres kératiniques pendant une durée suffisante pour développer la coloration désirée.

## Patentansprüche

1. Zusammensetzung zum oxidativen Färben von Keratinfasern, die Folgendes umfasst: A) mindestens 25 Gew.-%, bezogen auf das Gesamtgewicht der Zusammensetzung, von einem oder mehreren Fettkörper(n), wobei mindestens einer der Fettkörper aus Fettsäureamiden und Fettsäureestern, die bei einer Temperatur von 25°C und Atmosphärendruck (760 mm Hg) fest sind, ausgewählt ist und mindestens einer der Fettkörper aus Fettkörpern, die bei einer Temperatur von 25°C und Atmosphärendruck (760 mm Hg) flüssig sind, ausgewählt ist, B) eine oder mehrere Vorstufen von Färbemitteln, C) ein oder mehrere Oxidationsmittel und gegebenenfalls D) ein oder mehrere alkalische Mittel.

2. Zusammensetzung nach Anspruch 1, **dadurch gekennzeichnet, dass** die Fettsäureamide Amide eines gegebenenfalls substituierten C2-C10-Mono- oder Dialkanolamins und einer C9-C30-Fettsäure sind.

3. Zusammensetzung nach einem der vorhergehenden Ansprüche, wobei das Fettsäureamid der nachstehenden Formel (I) entspricht: 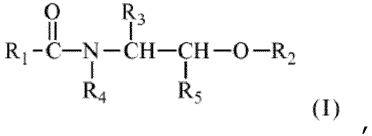 worin:
- R₁ entweder für einen geraden oder verzweigten, gesättigten oder ungesättigten C₉-C₃₀-Kohlenwasserstoffrest, wobei dieser Rest mit einer oder mehreren Hydroxylgruppen substituiert sein kann, wobei diese Hydroxylgruppen gegebenenfalls mit einer gesättigten oder ungesättigten C₁₆-C₃₀-Fettsäure verestert sind; oder für einen Rest R"-(NR-CO)-R' steht, wobei R für ein Wasserstoffatom oder einen mono- oder polyhydroxylierten, vorzugsweise monohydroxylierten C₁-C₁₀-Kohlenwasserstoffrest steht, R' und R" Kohlenwasserstoffreste sind, deren Summe der Kohlenstoffatome zwischen 9 und 30 beträgt, wobei R' ein zweiwertiger Rest ist;
- R₂ für ein Wasserstoffatom oder einen Rest (Glykosyl)ₙ, (Galactosyl)ₘ oder Sulfogalactosyl steht, wobei n eine ganze Zahl im Bereich von 1 bis einschließlich 4 ist und m eine ganze Zahl im Bereich von 1 bis einschließlich 8 ist;
- R₃ ein Wasserstoffatom oder einen gesättigten oder ungesättigten C₁₆-C₂₇-Kohlenwasserstoffrest bedeutet, wobei dieser Rest mit einem oder mehreren C₁-C₁₄-Alkylresten substituiert sein kann; wobei R₃ auch für einen α-Hydroxy-C₁₅-C₂₆-alkylrest stehen kann, dessen Hydroxylgruppe gegebenenfalls mit einer C₁₆-C₃₀)-α-Hydroxysäure verestert sein kann;
- R₄ ein Wasserstoffatom, einen gesättigten oder ungesättigten C₁₆-C₂₇-Kohlenwasserstoffrest, einen C2-C6-Hydroxyalkylrest oder einen Rest -CH₂-CHOH-CH₂-O-R₆, wobei R₆ für einen C₁₀-C₂₆-Kohlenwasserstoffrest steht, bedeutet;
- R₅ ein Wasserstoffatom oder einen mono- oder polyhydroxylierten C₁-C₄-Kohlenwasserstoffrest bedeutet.

4. Zusammensetzung nach Anspruch 3, wobei das Fettsäureamid ein Ceramid der Formel (I) ist, worin R₁ für einen gegebenenfalls hydroxylierten, gesättigten oder ungesättigten, von C₁₆-C₂₂-Fettsäuren stammenden Alkylrest steht; R₂ für ein Wasserstoffatom steht und R₃ für einen α-Hydroxy-C16-alkylrest steht.

5. Zusammensetzung nach einem der vorhergehenden Ansprüche, **dadurch gekennzeichnet, dass** das Fettsäureamid aus Stearinsäuremonoethanolamid, Monoisopropanolamid von Kokossäuren, Monoethanolamid von Kokossäuren und N-Oleoyldihydrosphingosin ausgewählt ist.

6. Zusammensetzung nach einem der vorhergehenden Ansprüche, wobei der Fettsäureester ausgehend von C8-C30-Fettsäuren erhalten wird.

7. Zusammensetzung nach Anspruch 6, wobei die Fettsäure aus Stearinsäure, Palmitinsäure, Laurinsäure, Ölsäure, Myristinsäure ausgewählt ist.

8. Zusammensetzung nach einem der vorhergehenden Ansprüche, wobei der Ester aus Ethylenglykoldistearat, Stearylstearat, -palmitat oder -myristat, Cetylstearat, -palmitat oder -myristat, Myristylstearat, -palmitat oder -myristat ausgewählt ist.

9. Zusammensetzung nach einem der vorhergehenden Ansprüche, **dadurch gekennzeichnet, dass** die Konzentration an Fettsäureamid und/oder Fettsäureester(n) im Allgemeinen zwischen 0,01 und 50 Gew.-%, insbesondere zwischen 0,1 und 30 Gew.-% und stärker bevorzugt zwischen 0,2 und 10 Gew.-%, bezogen auf das Gesamtgewicht der Zusammensetzung, beträgt.

10. Zusammensetzung nach einem der vorhergehenden Ansprüche, wobei der oder die flüssige(n) Fettkörper aus Vaselineöl, Polydecenen, Estern von Fettsäure und Fettalkohol oder deren Gemisch ausgewählt ist/sind.

11. Zusammensetzung nach einem der vorhergehenden Ansprüche, **dadurch gekennzeichnet, dass** die Färbemittelvorstufe aus Oxidationsbasen und Kupplern ausgewählt ist.

12. Zusammensetzung nach dem vorhergehenden Anspruch, **dadurch gekennzeichnet, dass** die Färbemittelvorstufe aus den Oxidationsbasen ortho- und para-Phenylendiamine, Doppelbasen, ortho- und para-Aminophenole, heterozyklischen Basen sowie Additionssalzen dieser Verbindungen mit einer Säure und aus den Kupplern meta-Aminophenole, meta-Phenylendiamine, meta-Diphenole, Naphthole, heterozyklischen Kupplern und den Additionssalzen dieser Verbindungen mit einer Säure ausgewählt ist.

13. Zusammensetzung nach einem der vorhergehenden Ansprüche, **dadurch gekennzeichnet, dass** das Oxidationsmittel ein Peroxid, vorzugsweise Wasserstoffperoxid ist.

14. Zusammensetzung nach einem der vorhergehenden Ansprüche, wobei das alkalische Mittel Monoethanolamin ist.

15. Verfahren zum Färben von Keratinfasern, **dadurch gekennzeichnet, dass** eine Zusammensetzung nach einem der Ansprüche 1 bis 14 auf die Keratinfasern für einen Zeitraum aufgebracht wird, der zur Entwicklung der gewünschten Färbung ausreicht.

## Claims

1. Composition for the oxidation dyeing of keratin fibres, comprising A) at least 25% by weight of the total weight of the composition of one or more fatty substances, at least one of the fatty substances being selected from the fatty amides and the esters of fatty acid which are solid at a temperature of 25°C and at atmospheric pressure (760 mmHg) and at least one of the fatty substances being selected from the fatty substances which are liquid at a temperature of 25°C and at atmospheric pressure (760 mmHg), B) one or more dye precursors, C) one or more oxidizing agents and D) one or more alkaline agents, in which the alkaline agent is an alkanolamine.

2. Composition according to Claim 1, **characterized in that** the fatty amides are amides of a C₂-C₁₀ mono- or dialkanolamine, optionally substituted, and of a C₉-C₃₀ fatty acid.

3. Composition according to either one of the preceding claims, in which the fatty amide corresponds to the following formula (I): 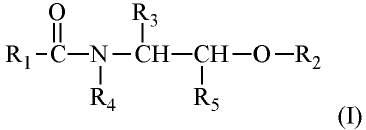 in which:
- R₁ denotes either a linear or branched, saturated or unsaturated C₉-C₃₀ hydrocarbon radical, which can be substituted with one or more hydroxyl groups, said hydroxyl groups being optionally esterified by a saturated or unsaturated C₁₆-C₃₀ fatty acid; or a radical R"-(NR-CO)-R', where R denotes a hydrogen atom or a mono- or polyhydroxylated, preferably monohydroxylated, C₁-C₁₀ hydrocarbon radical, R' and R" are hydrocarbon radicals in which the total number of carbon atoms is between 9 and 30, R' being a divalent radical;
- R₂ denotes a hydrogen atom or a (glycosyl)ₙ, (galactosyl)ₘ or sulfogalactosyl radical, in which n is an integer in the range from 1 to 4 inclusive and m is an integer in the range from 1 to 8 inclusive;
- R₃ denotes a hydrogen atom or a saturated or unsaturated C₁₆-C₂₇ hydrocarbon radical, which can be substituted with one or more C₁-C₁₄ alkyl radicals; R₃ can also denote a C₁₅-C₂₆ α-hydroxyalkyl radical whose hydroxyl group can optionally be esterified by a C₁₆-C₃₀ α-hydroxyacid;
- R₄ denotes a hydrogen atom, a saturated or unsaturated C₁₆-C₂₇ hydrocarbon radical, a C₂-C₆ hydroxyalkyl radical or a radical -CH₂-CHOH-CH₂-O-R₆ in which R₆ denotes a C₁₀-C₂₆ hydrocarbon radical;
- R₅ denotes a hydrogen atom or a mono- or polyhydroxylated C₁-C₄ hydrocarbon radical.

4. Composition according to Claim 3, in which the fatty amide is a ceramide of formula (I), in which R₁ denotes a saturated or unsaturated alkyl radical derived from C₁₆-C₂₂ fatty acids, optionally hydroxylated; R₂ denotes a hydrogen atom; and R₃ denotes a C₁₆ α-hydroxyalkyl radical.

5. Composition according to any one of the preceding claims, **characterized in that** the fatty amide is selected from the monoethanolamide of stearic acid, the monoisopropanolamide of copra acids, the monoethanolamide of copra acids and N-oleoyldihydrosphingosine.

6. Composition according to any one of the preceding claims, in which the ester of fatty acid is obtained from C₈-C₃₀ fatty acids.

7. Composition according to Claim 6, in which the fatty acid is selected from stearic acid, palmitic acid, lauric acid, oleic acid, myristic acid.

8. Composition according to any one of the preceding claims, in which the ester is selected from ethylene glycol distearate, stearyl stearate, palmitate or myristate, cetyl stearate, palmitate or myristate, myristyl stearate, palmitate or myristate.

9. Composition according to any one of the preceding claims, **characterized in that** the concentration of fatty amide and/or fatty acid ester(s) is generally between 0.01 and 50 wt.%, in particular between 0.1 and 30 wt.%, and more preferably between 0.2 and 10 wt.%, relative to the total weight of the composition.

10. Composition according to any one of the preceding claims, in which the liquid fatty substances are selected from liquid paraffin, polydecenes, esters of fatty acid and fatty alcohol or a mixture thereof.

11. Composition according to any one of the preceding claims, **characterized in that** the dye precursor is selected from oxidation bases and couplers.

12. Composition according to the preceding claim, **characterized in that** the dye precursor is selected from the ortho- and para-phenylenediamine oxidation bases, the double bases, the ortho- and para-aminophenols, the heterocyclic bases, as well as the salts of addition of these compounds with an acid and the couplers meta-aminophenols, meta-phenylenediamines, meta-diphenols, naphthols, heterocyclic couplers and salts of addition of these compounds with an acid.

13. Composition according to any one of the preceding claims, **characterized in that** the oxidizing agent is a peroxide, preferably hydrogen peroxide.

14. Composition according to any one of the preceding claims, in which the alkaline agent is monoethanolamine.

15. Method of dyeing of keratin fibres, **characterized in that** a composition as defined in any one of Claims 1 to 14 is applied on the keratin fibres for a sufficient time to develop the desired coloration.
